# EUROPÄISCHE PATENTSCHRIFT

(11) **EP 0 482 157 B1**
(45) Veröffentlichungstag und Bekanntmachung des Hinweises auf die Patenterteilung: **17.11.1999**
(21) Anmeldenummer: 91908750.2
(22) Anmeldetag: 24.04.1991
(51) Int. Cl.: C07D 319/06, C09K 19/34

(54) **1,3-DIOXANDERIVATE UND FLÜSSIGKRISTALLINES MEDIUM**
1,3-DIOXANE DERIVATES AND LIQUID CRYSTAL MEDIUM
DERIVES D'1,3-DIOXANE ET MILIEU DE CRISTAUX LIQUIDES

(30) Priorität: 26.04.1990 DE 4013242
(43) Veröffentlichungstag der Anmeldung: 29.04.1992
(73) Patentinhaber: MERCK PATENT GmbH, 64271 Darmstadt (DE)
(72) Erfinder: REIFFENRATH, Volker, D-6101 Rossdorf (DE); KRAUSE, Joachim, D-6110 Dieburg (DE); HITTICH, Reinhard, D-6101 Modautel 1 (DE); POETSCH, Eike, D-6109 Mühltal 6 (DE); PLACH, Herbert, D-6100 Darmstadt (DE)
(86) Internationale Anmeldenummer: EP9100793
(87) Internationale Veröffentlichungsnummer: WO9116321

(56) Entgegenhaltungen:
- EP-A- 0 058 981
- EP-A- 0 087 679
- EP-A- 0 090 183
- EP-A- 0 122 389
- EP-A- 0 149 209
- EP-A- 0 154 840
- EP-A- 0 268 198
- EP-A- 0 269 963
- EP-A- 0 315 014
- EP-A- 0 387 032
- WO-A-86/00481
- WO-A-87/06602
- WO-A-88/02108
- WO-A-89/02884
- DE-A- 3 437 935
- DE-A- 4 027 840
- GB-A- 2 044 767
- GB-A- 2 067 586
- US-A- 4 325 830

## Beschreibung

Die Erfindung betrifft neue 1,3-Dioxanderivate der Formel I 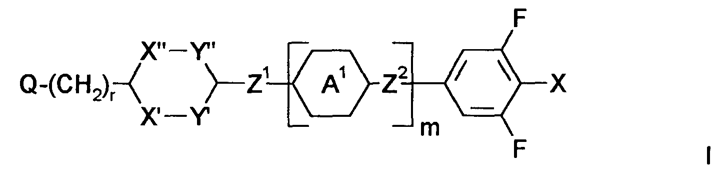 worin
- Q: CHalogenₚ H₃₋ₚ oder CₙH₂ₙ₊₁-O- oder CₙH₂ₙ₊₁-CH=CH-, wobei
- Halogen: F oder Cl,
- p: 1, 2 oder 3, und
- n: 0 bis 5 bedeutet,
- r: 0 bis 5,
X" = X'= O und Y" = Y' = CH₂ oder
und Y"=Y'=O, X" = X' = CH₂
- Z¹ und Z²: jeweils unabhängig voneinander -C₂H₄- oder eine Einfachbindung,
- A¹: trans-1,4-Cyclohexylen, 1,4-Phenylen, 3-Fluor-1 ,4-phenylen oder 3,5-Difluor-1,4-phenylen,
- m: 0,1 oder 2, und
- X: F, Cl, -CF₃, -CN, -OCF₃ oder -OCHF₂ bedeuten,
mit den Maßgaben, daß
a) im Falle X = F m = 1 oder 2 ist, und
b) im Falle X = F und m = 1 Z² -C₂H₄- bedeutet und/oder A¹ trans-1,4-Cyclohexylen, 3-Fluor-1,4-phenylen oder 3,5-Difluor-1,4-phenylen bedeutet.

Aus der DE-OS 29 44 905 sind Flüssigkristalle der Formel 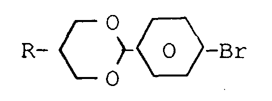 bekannt (R = C₆H₁₃, C₈H₁₇).

Derartige Verbindungen werden jedoch nicht den hohen Anforderungen an den elektrischen Widerstand gerecht, wie sie beispielsweise für Anzeigen mit aktiver Matrix gefordert werden. Darüberhinaus zeigen solche Verbindungen in flüssigkristallinen Medien recht hohe Werte für die Schwellenspannung.

Die Verbindungen der Formel I können wie ähnliche, z.B. aus der DE-OS 26 36 684 bekannte Verbindungen als Komponenten flüssigkristalliner Medien verwendet werden, insbesondere für Anzeigen, die auf dem Prinzip der verdrillten Zelle beruhen.

1,3-Dioxanderivate mit fluorierten Phenylringen werden in der EP-A-154 840 sowie in der nicht vorpublizierten EP-A-387 032 beschrieben.

Die bisher für diesen Zweck eingesetzten Substanzen besitzen sämtliche gewisse Nachteile, beispielsweise zu hohe Schmelzpunkte, zu niedrige Klärpunkte, zu geringe Stabilität gegenüber der Einwirkung von Wärme, Licht oder elektrischen Feldern, zu niedriger elektrischer Widerstand, ungünstige elastische Eigenschaften, zu hohe Temperaturabhängigkeit der Schwellenspannung oder führen zu zu hohen Schwellenspannungen.

Insbesondere bei Anzeigen von Supertwisttyp (STN) mit Verdrillungswinkeln von deutlich mehr als 220 °C oder bei Anzeigen mit aktiver Matrix weisen die bisher eingesetzten Materialien Nachteile auf.

Der Erfindung lag die Aufgabe zugrunde, neue flüssigkristalline Verbindungen aufzufinden, die als Komponenten flüssigkristalliner Medien geeignet sind, insbesondere für nematische Medien mit positiver dielektrischer Anisotropie, und die die Nachteile der bekannten Verbindungen nicht oder nur in geringerem Maße zeigen. Diese Aufgabe wurde durch die Bereitstellung der neuen Verbindungen der Formel I gelöst.

Es wurde gefunden, daß die Verbindungen der Formel I vorzüglich als Komponenten flüssigkristalliner Medien geeignet sind. Insbesondere sind mit ihrer Hilfe flüssigkristalline Medien mit weiten nematischen Bereichen, hervorragender Nematogenität bis zu tiefen Temperaturen, hervorragender chemischer Stabilität, herausragenden elastischen Eigenschaften, ausgeprägtem ε^{┴} bei positiver dieelektrischer Anisotropie, niedriger Schwellenspannung, geringer Temperaturabhängigkeit der Schwellenspannung und/oder kleiner optischer Anisotropie erhältlich. Die neuen Verbindungen zeigen außerdem eine gute Löslichkeit für andere Komponenten derartiger Medien und hohe positive dielektrische Anisotropie bei gleichzeitig günstiger Viskosität.

Die Verbindungen der Formel I ermöglichen sowohl STN-Anzeigen mit sehr hoher Steilheit der elektrooptischen Kennlinie als auch Anzeigen mit aktiver Matrix mit hervorragender Langzeitstabilität. Durch geeignete Wahl von rund n lassen sich bei beiden Anzeigetypen die Schwellenspannungen deutlich erniedrigen.

Die Verbindungen der Formel I sind in reinem Zustand farblos und bilden flüssigkristalline Mesophasen in einem für die elektrooptische Verwendung günstig gelegenen Temperaturbereich.

Gegenstand der Erfindung sind somit die Verbindungen der Formel I sowie die Verwendung der Verbindungen der Formel I als Komponenten flüssigkristalliner Medien, flüssigkristalline Medien mit einem Gehalt an mindestens einer Verbindung der Formel I und elektrooptische Anzeigen, die derartige Medien enthalten.

Vor- und nachstehend haben Q, r, X", Y", X', Y', Z¹, Z², A¹, X, und m die angegebene Bedeutung, sofern nicht ausdrücklich etwas anderes vermerkt ist.

In den Verbindungen der Formel I sind die Alkylengruppen (CH₂)ᵣ vorzugsweise geradkettig. Dementsprechend bedeutet (CH₂)ᵣ vorzugsweise Methylen, Ethylen, n-Propylen, n-Butylen oder n-Pentylen. m ist vorzugsweise 0 oder 1, insbesondere bevorzugt 0. r ist vorzugsweise 2, 3 oder 4. Ferner bevorzugt ist r = 1, insbesondere wenn Q = CₙH₂ₙ₊₁-CH=CH-.

X ist vorzugsweise F, Cl, -CF₃ oder -OCF₃.

Im folgenden sind bevorzugte Teilformeln der erfindungsgemäßen Verbindungen angegeben:

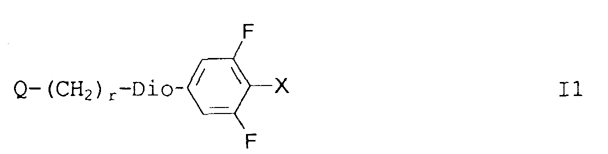

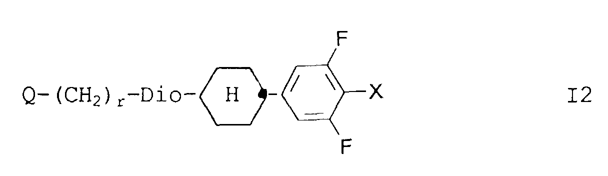

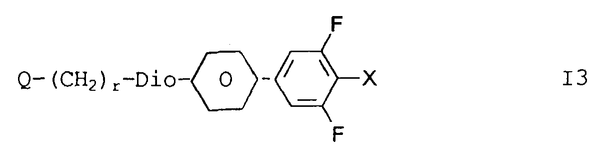

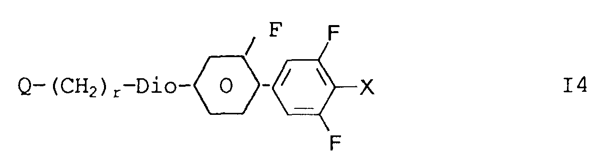

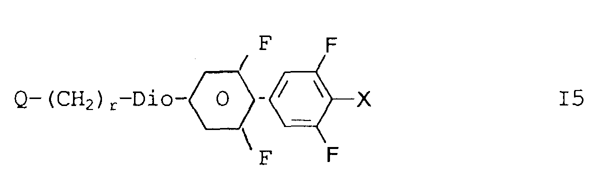

Z¹ und Z² sind bevorzugt Einfachbindungen oder eine dieser Gruppen auch -C₂H₄-. m ist vorzugsweise 0 oder 1. Ferner sind jedoch auch Verbindungen mit m = 2 (wobei A¹ und Z² gleich oder voneinander verschieden sind) bevorzugt, insbesondere diejenigen der folgenden Teilformeln

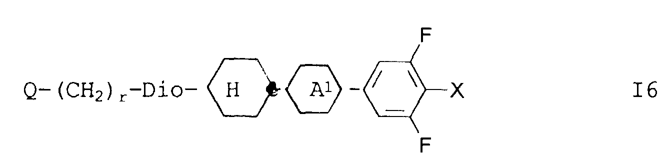

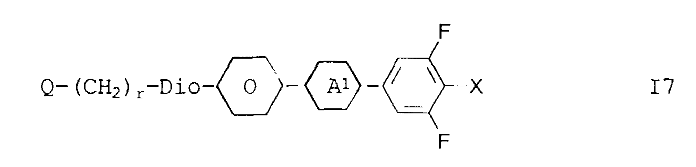

Besonders bevorzugt sind die Verbindungen der Teilformel Ia 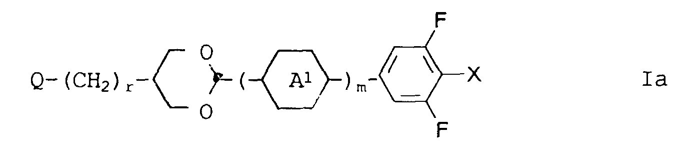 worin Q, r, A¹, m, X, die bei der Formel I angegebene Bedeutung haben. A¹ ist vorzugsweise trans-1,4-Cyclohexylen. Q ist vorzugsweise CF₃, CHF₂, CH₂F, CH₂Cl, CH₃O-, CH₂=CH- oder CH₃-CH=CH-(trans). Halogen ist vorzugsweise F.

In den vorstehenden Formeln bedeutet Dio 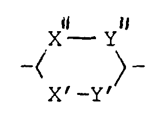 und ist vorzugsweise

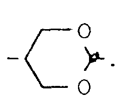

Die Verbindungen der Formel I werden im übrigen nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Die erfindungsgemäßen 1,3-Dioxanderivate werden in an sich üblicher Weise durch Kondensation von 1,3-Diolen der Formel IIa mit Aldehyden der Formel IIIa bzw. von 1,3-Diolen der Formel IIb mit Aldehyden der Formel IIIb hergestellt:

Q-(CH₂)ᵣ-CH(CH₂OH)₂ IIa

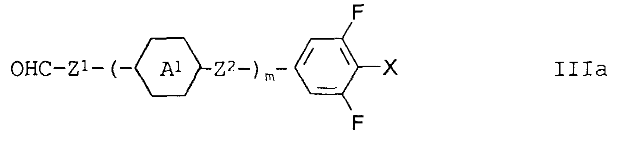

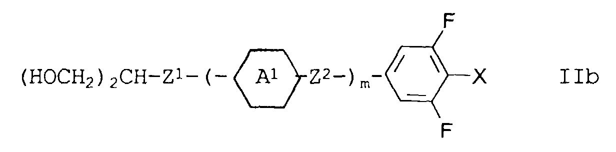

Q-(CH₂)ᵣ-CHO IIIb

Die Vorstufen der Formeln IIa, IIIa, IIb und IIIb sind teilweise bekannt. Alle diese Verbindungen können nach Routinemethoden hergestellt werden, beispielsweise nach folgendem Syntheseschema:

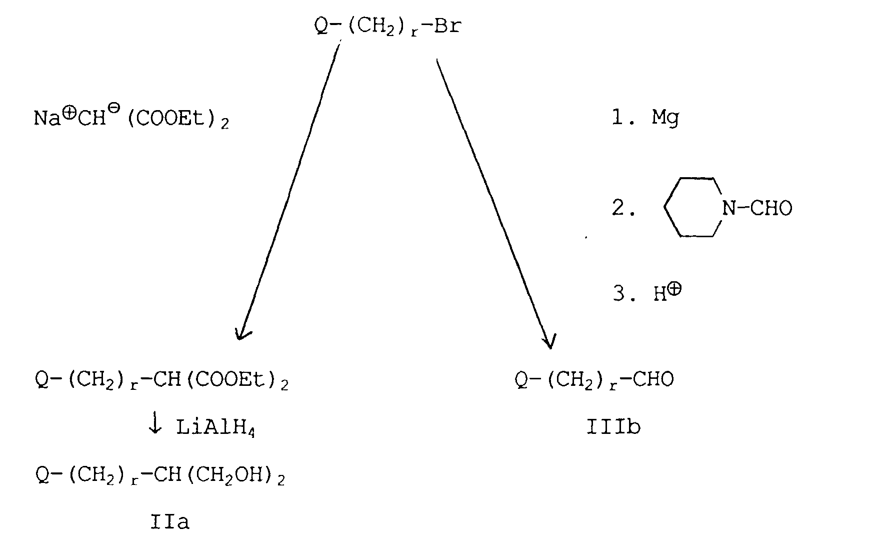

Die Verbindungen der Formeln IIIa und IIb können analog hergestellt werden. Die Synthese einiger besonders bevorzugter Verbindungen der Formeln IIIa und IIb sind im folgenden näher erläutert

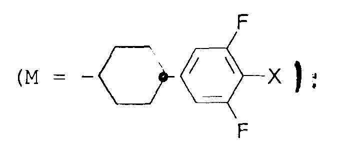

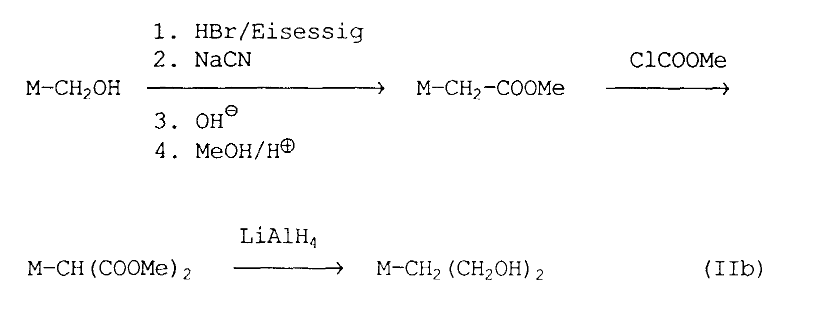

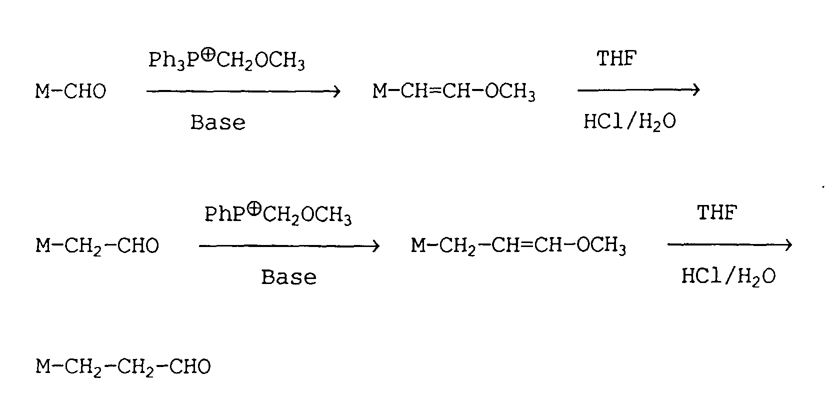

Verbindungen der Formel IIIa sind ferner durch Übergangsmetallkatalysierte Kreuzkopplungsreaktionen (E. Poetsch, Kontakte (Darmstadt) 1988 (2), S. 15) erhältlich, wie z.B.:

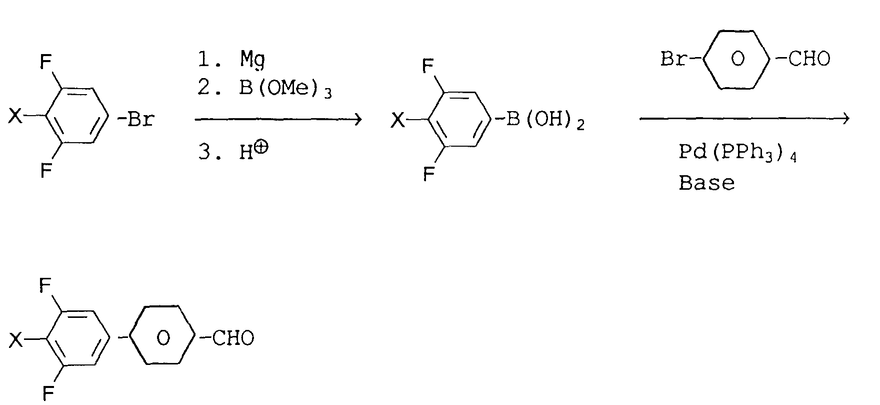

Zur Synthese der erfindungsgemäßen Verbindungen der Formel I2 geeignete Vorstufen sind beispielsweise nach folgendem Syntheseschema erhältlich:

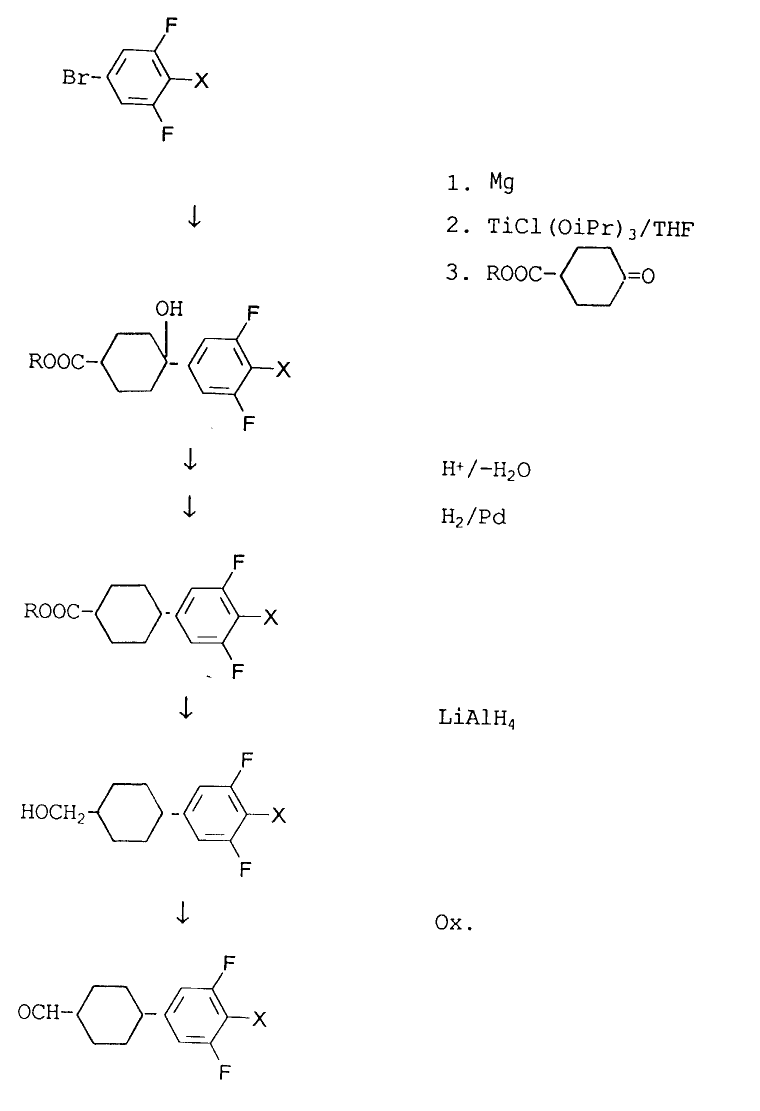

Die aus dem entsprechenden Brombenzol-Derivat erhaltene Grignard-Verbindung wird mit Chlortrialkylorthotitanat bzw. -zirkonat nach WO 87/05599 zu dem tertiären Cyclohexanol umgesetzt. Nach Abspaltung von Wasser, Hydrierung der Doppelbindung und Isomerisierung erhält man nach üblichen Methoden den trans-Cyclohexancarbonsäureester. Aus letzterem erhält man nach üblichen Standardverfahren die geeigneten Aldehyde für die erfindungsgemäßen Verbindungen, die aus diesen durch Wittig-Synthese und nachfolgender Hydrierung der Doppelbindung erhältlich sind.

Die als Ausgangsstoffe verwendete Brombenzolderivate sind zum Teil bekannt, zum Teil können sie ohne Schwierigkeiten nach Standardverfahren der organischen Chemie aus literaturbekannten Verbindungen hergestellt werden. Beispielsweise sind die OCF₃- oder OCHF₂-Verbindungen nach bekannten Verfahren aus den entsprechenden Phenolen bzw. die CF₃- oder CN-Verbindungen aus den entsprechenden Benzoesäuren erhältlich. Verbindungen der Formel 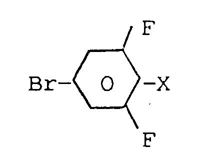 oder auch entsprechende monofluorierte Verbindungen sind beispielsweise aus den bekannten Vorstufen mit X = H durch Lithiierung bei tiefen Temperaturen und anschließende Umsetzung mit einem geeigneten Elektrophil erhältlich.

Die Synthese eines besonders bevorzugten Ausgangsmaterials der Formel IIIa' ist im folgenden beschrieben:

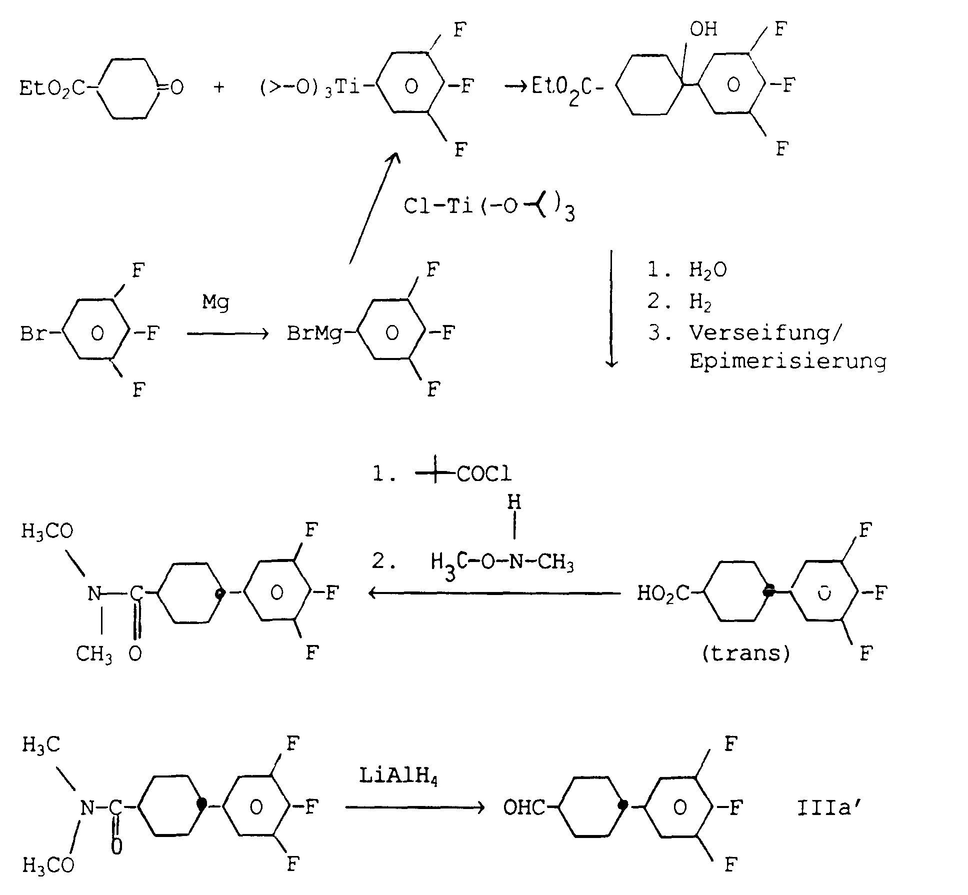

Die Kondensation dieses Zwischenprodukts mit Verbindungen der Formel IIa liefert bevorzugte Dioxanderivate. Q ist vorzugsweise CH₂=CH-. Die entsprechenden Verbindungen der Formel IIa erhält man vorzugsweise gemäß folgendem Schema:

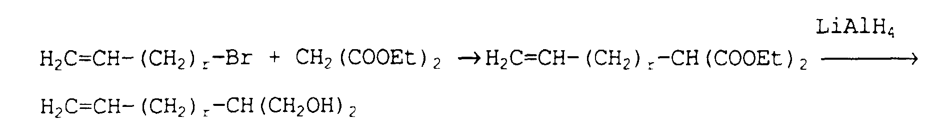

Besonders bevorzugt sind auch Verbindungen der Teilformel 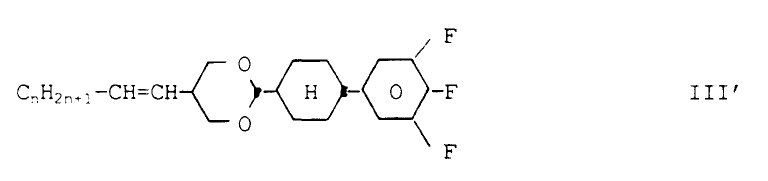 welche gemäß folgendem Schema hergestellt werden können:

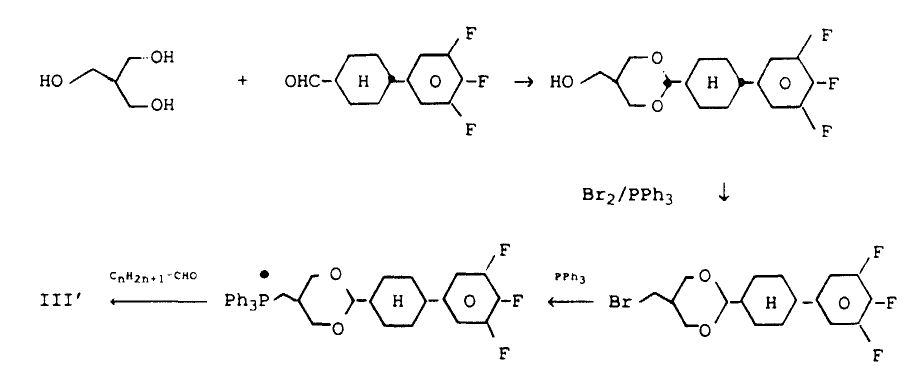

Die Alkylierung des Malonesters gelingt in bekannter Weise durch die Erzeugung seines Anions und Umsetzung mit den Alkenylbromiden. Die Reduktion zu den Diolen erfolgt in üblicher Weise durch Reduktion mit LiAlH4. Die Kondensation der erhaltenen Diole mit dem Aldehyd IIIa' erfolgt ebenfalls in analoger Weise zur Bildung bereits bekannter Dioxansysteme durch Kochen äquimolarer Mengen des Diols mit dem Aldehyd in Toluol am Wasserabscheider unter Zusatz von p-Toluolsulfonsäure. Die Reaktion ist bereits nach 4 Std. beendet.

Das Reaktionsprodukt wird durch eine Chromatographie uDer Kieselgel und Kristallisation aus Hexan aufgereinigt. Auch die Synthese des benötigten Aldehyds IIIa' erfolgt nach bekannten Methoden.

So wird 1-Trisopropyloxytitanyl, 3,4,5-trifluorbenzol - hergestellt durch Ummetallierung von 3,4,5-Trifluorbenzolmagnesiumbromid mit Chlortriisoproxytitan - mit Cyclohexanon-(4)-carbonsäureethylester-(1) chemoselektiv an der Carbonylgruppe zum 4-Hydroxy-4(3,4, 5-trifluorphenyl)-cyclohexancarbonsäureethylester umgesetzt. Durch Wasserabspaltung, katalytische Hydrierung über Pd/C, Verseifung und Epimerisierung mit KOH/Methanol erhält man die reine trans 4-(3,4,5-Trifluorphenyl)-cyclohexancarbonsäure-(1). Diese wird mit Pivaloylchlorid in das gemische Anhydrid umgewandelt und mit N,O-Dimethylhydroxylamin in das entsprechende Amid übergeführt. Dieses wird in hoher Ausbeute zum Aldehyd IIIa' mit LiAlH₄ reduziert.

Die erfindungsgemäßen flüssigkristallinen Medien enthalten vorzugsweise neben einer oder mehreren erfindungsgemäßen Verbindungen als weitere Bestandteile 2 bis 40, insbesondere 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Biphenyle, Terphenyle, Phenyl- oder Cyclohexylbenzoate, Cyclohexan-carbonsäurephenyl- oder cyclohexyl-ester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure, bzw. der Cyclohexylcyclohexancarbonsaure, Phenylcyclohexane, Cyclohexylbiphenyle, Phenylcyclohexylcyclohexane, Cyclohexylcyclohexane, Cyclohexylcyclohexene, Cyclohexylcyclohexylcyclohexene, 1,4-Bis-cyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane, Phenyl-oder Cyclohexyl-1,3-dithiane, 1,2-Diphenylethane, 1,2-Dicyclohexylethane, 1-Phenyl-2-cyclohexylethane, l-Cyclohexyl-2-(4-phenylcyclohexyl)-ethane, 1-Cyclohexyl-2-biphenylylethane, 1-Phenyl-2-cyclohexylphenylethane und Tolane.

Die 1,4-Phenylengruppen in diesen Verbindungen können auch fluoriert sein.

Die wichtigsten als weitere Bestandteile erfindungsgemäßer Medien in Frage kommenden Verbindungen lassen sich durch die Formeln 1, 2, 3, 4 und 5 charakterisieren:

R'-L-E-R" 1

R'-L-COO-E-R" 2

R'-L-OOC-E-R" 3

R'-L-CH₂CH₂-E-R" 4

R'-L-CC-E-R" 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten.

Vorzugsweise ist einer der Reste L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und R" bedeuten in den Verbindungen der Teilformeln la, 2a, 3a, 4a und 5a jeweils unabhängig voneinander Alkyl, Alkenyl, Alkoxy, Alkenyloxy oder Alkanoyloxy mit bis zu 8 Kohlenstoffatomen. Bei den meisten dieser Verbindungen sind R' und R" voneinander verschieden, wobei einer dieser Reste meist Alkyl oder Alkenyl ist. In den Verbindungen der Teilformeln 1b, 2b, 3b, 4b und 5b bedeutet R" -CN, -CF₃, -OCF₃, F, Cl oder -NCS; R hat dabei die bei den Verbindungen der Teilformeln la bis 5a angegebene Bedeutung und ist vorzugsweise Alkyl oder Alkenyl. Besonders bevorzugt ist R" ausgewählt aus der Gruppe bestehend aus -F, Cl, CF₃ und -OCF₃. Aber auch andere Varianten der vorgesehenen Substituenten in den Verbindungen der Formeln 1, 2, 3, 4 und 5 sind gebräuchlich. Viele solcher Substanzen oder auch Gemische davon sind im Handel erhältlich. Alle diese Substanzen sind nach literaturbekannten Methoden oder in Analogie dazu erhältlich.

Die erfindungsgemäßen Medien enthalten vorzugsweise neben Komponenten aus der Gruppe der Verbindungen 1a, 2a, 3a, 4a und 5a (Gruppe 1) auch Komponenten aus der Gruppe der Verbindungen 1b, 2b, 3b, 4b und 5b (Gruppe 2), deren Anteile vorzugsweise wie folgt sind:
Gruppe 1: 20 bis 90 %, insbesondere 30 bis 90 %,
Gruppe 2: 10 bis 80 %, insbesondere 10 bis 50 %,
wobei die Summe der Anteile der erfindungsgemäßen Verbindungen und der Verbindungen aus den Gruppen 1 und 2 bis zu 100 % ergeben.

Die erfindungsgemäßen Medien enthalten vorzugsweise 1 bis 40 %, insbesondere vorzugsweise 5 bis 30 % an erfindungsgemäßen Verbindungen. Weiterhin bevorzugt sind Medien, enthaltend mehr als 40 %, insbesondere 45 bis 90 % an erfindungegemäßen Verbindungen. Die Medien enthalten vorzugsweise drei, vier oder fünf erfindungsgemäße Verbindungen.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Die folgenden Beispiele sollen die Erfindung erläutern, ohne sie zu begrenzen. mp. = Schmelzpunkt, cp. = Klärpunkt. Vor- und nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

Es bedeuten ferner:
K: Kristallin-fester Zustand, S: smektische Phase (der Index kennzeichnet den Phasentyp), N: nematischer Zustand, Ch; cholesterische Phase, I: isotrope Phase. Die zwischen zwei Symbolen stehende Zahl gibt die Umwandlungstemperatur in Grad Celsius an.
- DAST: Diethylaminoschwefeltrifluorid
- DCC: Dicyclohexylcarbodiimid
- DDQ: Dichlordicyanobenzochinon
- DIBALH: Diisobutylaluminiumhydrid
- DMSO: Dimethylsulfoxid
- KOT: Kalium-tertiär-butanolat
- THF: Tetrahydrofuran
- pTSOH: p-Toluolsulfonsäure

### Beispiel 1

Ein Gemisch von 0,1 m 2-(3-Fluoropropyl)-1,3-propandiol, 0,1 m p-(3,4-Difluorphenyl)-benzaldehyd (hergestellt durch Kreuzkopplung wie oben beschrieben), 1 g p-Toluolsulfonsäure und 200 ml Toluol wird 1 Stunde am Wasserabscheider gekocht. Nach extraktiver Aufarbeitung und Aufreinigung durch Kristallisation und Chromatographie erhält man trans-2-(3,4-Difluorbiphenyl-4'-yl)-5-(3-fluorpropyl)-1,3-dioxan.

Analog bzw. nach den obigen Syntheseschemata werden die folgenden bevorzugten Verbindungen hergestellt (X" = X' = CH₂, Y" = Y' = O, Z¹ = Z² = Einfachbindung, A¹ = trans-1,4-Cyclohexylen, m = 1):

| Q | r | X | Y | Z* |
|---|---|---|---|---|
| H₂C=CH- | 0 | F | F | F |
| H₂C=CH- | 1 | F | F | F |
| H₂C=CH- | 2 | F | F | F |
| CH₃CH=CH- | 0 | F | F | F |
| CH₃CH=CH- | 1 | F | F | F |
| CH₃CH=CH- | 2 | F | F | F |
| CF3 | 1 | F | F | F |
| CF₃ | 2 | F | F | F |
| CF3 | 3 | F | F | F |
| CF₃ | 4 | F | F | F |
| CF3 | 5 | F | F | F |
| CHF₂ | 2 | F | F | F |
| CHF₂ | 3 | F | F | F |
| CHF₂ | 4 | F | F | F |
| CHF₂ | 5 | F | F | F |
| CH₃O | 2 | F | F | F |
| CH₃O | 3 | F | F | F |
| CH₃O | 4 | F | F | F |
| CH₃O | 5 | F | F | F |

## Patentansprüche

1. 1,3-Dioxanderivate der Formel I 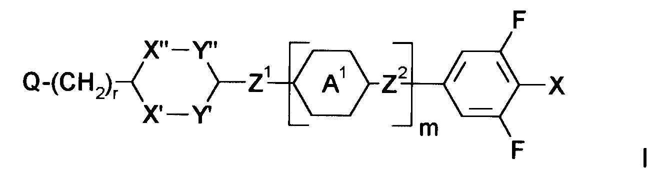 worin
Q CHalogenₚ H₃₋ₚ oder CₙH₂ₙ₊₁-O- oder CₙH₂ₙ₊₁-CH=CH-, wobei
Halogen F oder Cl,
p 1, 2 oder 3, und
n 0 bis 5 bedeutet,
r 0 bis 5,
X" = X' = O und Y" = Y' = CH₂ oder
X" = X' = CH₂ und Y" = Y' = O,
Z¹ und Z² jeweils unabhängig voneinander -C₂H₄- oder eine Einfachbindung,
A¹ trans-1,4-Cydohexylen, 1,4-Phenylen, 3-Fluor-1,4-phenylen oder 3,5-Difluor-1,4-phenylen,
m 0,1 oder 2, und
X F, Cl, -CF₃, -CN, -OCF₃ oder -OCHF₂ bedeuten,
mit den Maßgaben, daß
a) im Falle X = F m = 1 oder 2 ist, und
b) im Falle X = F und m = 1 Z² -C₂H₄- bedeutet und/oder A¹ trans-1,4-Cydohexylen, 3-Fluor-1,4-phenylen oder 3,5-Difluor-1,4-phenylen bedeutet.

2. 1,3-Dioxanderivate nach Anspruch 1, gekennzeichnet durch die Formel la 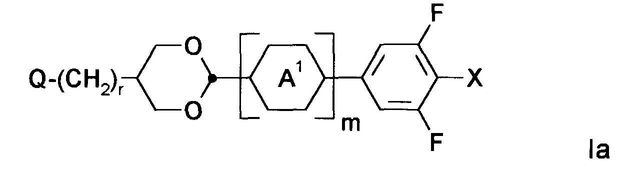 worin Q, r, A¹, m und X die in Formel I angegebene Bedeutung besitzen.

3. 1,3-Dioxanderivate nach Anspruch 2, dadurch gekennzeichnet, daß Q -CH₂Halogen, -OCH₃ oder -CH=CH₂ bedeutet, wobei Halogen F oder Cl ist.

4. Verwendung der 1,3-Dioxanderivate nach einem der Ansprüche 1 bis 3 als Komponenten flüssigkristalliner Medien für elektrooptische Anzeigen.

5. Flüssigkristallines Medium für elektrooptische Anzeigen mit mindestens zwei flüssigkristallinen Komponenten, daurch gekennzeichnet, daß mindestens eine Komponente ein 1,3-Dioxanderivat nach einem Ansprüche 1 bis 3 ist.

6. Elektrooptische Anzeige auf der Basis einer Flüssigkristallzelle, dadurch gekennzeichnet, daß die Flüssigkristallzelle ein Medium nach Anspruch 5 enthält.

## Claims

1. 1,3-Dioxane derivatives of the formula I 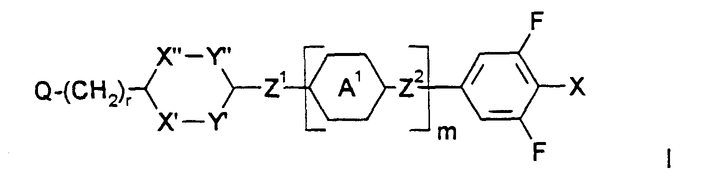 in which
Q is ChalogenₚH₃₋ₚ or CₙH₂ₙ₊₁-O- or CₙH₂ₙ₊₁-CH=CH-, where
Halogen is F or Cl,
p is 1, 2 or 3, and
n is 0 to 5,
r is 0 to 5,
X" = X' = O and Y" = Y' = CH₂ or
X" = X' = CH₂ and Y" = Y' = O,
Z¹ and Z² are each, independently of one another, -C₂H₄- or a single bond,
A¹ is trans-1,4-cyclohexylene, 1,4-phenylene, 3-fluoro-1,4-phenylene or 3,5-difluoro-1,4-phenylene,
m is 0, 1 or 2, and
X is F, Cl, -CF₃, -CN, -OCF₃ or -OCHF₂,
with the provisos that
a) in the case where X = F, m = 1 or 2, and
b) in the case where X = F and m = 1, Z² is -C₂H₄- and/or A¹ is trans-1,4-cyclohexylene, 3-fluoro-1,4-phenylene or 3,5-difluoro-1,4-phenylene.

2. 1,3-Dioxane derivatives according to Claim 1, characterized by the formula Ia 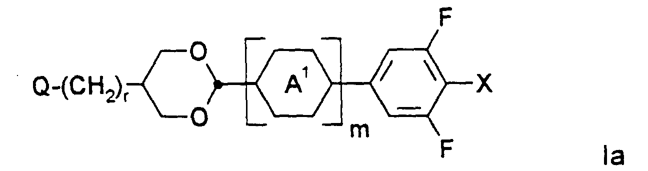 in which Q, r, A¹, m and X are as defined in formula I.

3. 1,3-Dioxane derivatives according to Claim 2, characterized in that Q is -CH₂Halogen, -OCH₃ or -CH=CH₂, Halogen being F or Cl.

4. Use of the 1,3-dioxane derivatives according to one of Claims 1 to 3 as components of liquid-crystalline media for electrooptical displays.

5. Liquid-crystalline medium for electrooptical displays containing at least two liquid-crystalline components, characterized in that at least one component is a 1,3-dioxane derivative according to one of Claims 1 to 3.

6. Electrooptical display based on a liquid-crystal cell, characterized in that the liquid-crystal cell contains a medium according to Claim 5.

## Revendications

1. Dérivés du 1,3-dioxanne de formule I 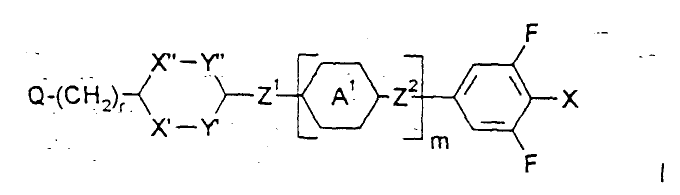 dans laquelle
Q représente ChalogèneₚH₃₋ₚ ou CₙH₂ₙ₊₁-O- ou CₙH₂ₙ₊₁-CH=CH-, dans lesquels
l'halogène est F ou Cl,
p est égal à 1, 2 ou 3 et
n est un nombre allant de 0 à 5,
r est un nombre allant de 0 à 5,
X" = X' = 0 et Y" = Y' = CH₂ ou
X" = X' = CH₂ et Y" = Y' = 0,
Z¹ et Z² représentent chacun, indépendamment l'un de l'autre, -C₂H₄- où une liaison simple,
A¹ représente un groupe trans-1,4-cyclohexylène, 1,4-phénylène, 3-fluoro-1,4-phénylène ou 3,5-difluoro-1,4-phénylène,
m est égal à 0, 1 ou 2 et
X représente F, Cl, CF₃, -CN, -OCF₃ ou -OCHF₂,
sous réserve que,
a) lorsque X = F, m est égal à 1 ou 2 et
b) lorsque X = 7 et que m est égal à 1, Z² représente -C₂H₄- et/ou A¹ représente un groupe trans-1,4-cyclohexylène, 3-fluoro-1,4-phénylène ou 3,5-difluoro-1,4-phénylène.

2. Dérivés du 1,3-dioxanne selon la revendication 1, caractérisés en ce qu'ils répondent à la formule Ia 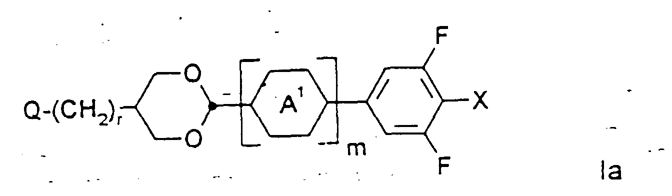 dans laquelle Q, r, A¹, m et X ont les significations indiquées en référence à la formule I.

3. Dérivés du 1,3-dioxanne selon la revendication 2, caractérisés en ce que Q représente -CH₂halogène, -OCH₃ ou -CH=CH₂, l'halogène étant F ou Cl.

4. Utilisation des dérivés du 1,3-dioxanne selon l'une des revendications 1 à 3 en tant que composants de milieux à cristaux liquides pour des affichages électro-optiques.

5. Milieu à cristaux liquides pour affichages électro-optiques, à au moins deux composants à cristaux liquides, caractérisé en ce que l'un au moins des composants est un dérivé du 1,3-dioxanne selon l'une des revendications 1 à 3.

6. Affichages électro-optiques à base d'une cellule à cristaux liquides, caractérisés en ce que la cellule à cristaux liquides contient un milieu selon la revendication 5.
